# FASCICULE DE BREVET EUROPEEN

(11) **EP 0 338 065 B1**
(45) Date de publication et mention de la délivrance du brevet: **26.01.1994**
(21) Numéro de dépôt: 88909777.0
(22) Date de dépôt: 27.10.1988
(51) Int. Cl.: C07J 9/00, C07J 17/00, A61K 31/575

(54) **DERIVES DE LA 19-NOR PROGESTERONE, LEUR PREPARATION ET LEUR UTILISATION**
19-NOR-PROGESTERON-DERIVATE, IHRE HERSTELLUNG UND ANWENDUNG
DERIVATIVES OF 19-NORPROGESTERONE, THEIR PREPARATION AND THEIR USE

(30) Priorité: 27.10.1987 FR 8714806
(43) Date de publication de la demande: 25.10.1989
(73) Titulaire: LABORATOIRE THERAMEX, Monaco (MC)
(72) Inventeur: PIASCO, Alain, F-06100 Nice (FR); NASRAOUI, Mohamed, Nejib, F-06100 Nice (FR)
(74) Mandataire: Burtin, Jean-François (FR)
(86) Numéro de dépôt international: FR8800527
(87) Numéro de publication internationale: WO8903839

(56) Documents cités:
- FR-A- 2 077 877
- FR-A- 2 271 833
- GB-A- 924 981

## Description

La présente invention se rapporte à de nouveaux dérivés de la 19-nor progestérone dont la chaine latérale portée par le carbone 17, comporte trois atomes de carbone.

Elle a plus particulièrement pour objet des dérivés de la 19-nor progestérone dont le noyau stéroide porte sur le carbone en 6 un substituant méthyle, sur le carbone en 17 un radical hydroxyle libre, éthérifié ou estérifié et dont la chaine carbonée en 17-β est une chaine propanone-3-.

Elle a spécifiquement pour objet les nouveaux dérivés de la 19-nor progestérone de formule générale I 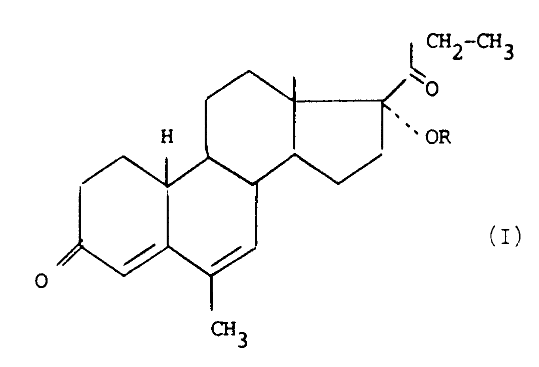
dans laquelle
R est un hydrogène, un radical alcoyle inférieur, méthoxyméthyle tétrahydropyranyle ou un reste acyle d'un acide organique carboxylique ou carbonique, aliphatique ou aromatique, ayant de 1 à 10 atomes de carbone.

Elle se rapporte en particulier aux composés suivants, actuellement préférés :
- le 17α-hydroxy 6,21 dimethyl 3,20-dioxo 19-nor pregna 4,6-diène
- le 17α-acetoxy 6,21-dimethyl 3,20-dioxo 19-nor pregna 4,6-diène
- le 17α-butyryloxy 6,21-diméthyl 3,20-dioxo 19-nor pregna 4,6-diène
- le 17α-tétrahydropyranyloxy 6,21-diméthyl 3,20-dioxo 19-nor pregna 4,6-diène
- le 17α-caproyloxy 6,21-diméthyl 3,20-dioxo 19-nor pregna 4,6-diène
- le 17α-heptanoyloxy 6,21-dimethyl 3,20-dioxo 19-nor pregna 4,6-diène
On connaissait déjà des dérivés de la 19-nor progestérone possédant un substituant 6-méthyle comme, par exemple, le Nomegestrol qui est un dérivé progestatif puissant et doué de propriétés progestéroniques pures.

Des dérivés de la 6-méthyl 3,20-dioxo 19-nor pregna-4,6-diène sont décrits dans le brevet français n^{o} 2271833.

On connaissait déjà, également, des dérivés de la 19-nor progestérone dont la chaine en 17 comportait 3 atomes de carbone et notamment le 3,20-dioxo 17α- 21-diméthyl 19-nor pregna 4,9-diène décrit dans le brevet français n^{o} 2.077.877. Ce type de composés ne manifeste que des propriétés progestomimétiques modestes par rapport à leur homologue inférieur.

Il a été trouvé, maintenant, que les composés de formule générale I possèdent des propriétés progestomimétiques tout à fait intéressantes, notamment par voie digestive et que leur liaison importante aux récepteurs de la progestérone implique une forte activité progestative.

L'efficacité de ces nouveaux composés peut être attribuée à la structure stéroide substituée par deux substituants volumineux, le méthyle en 6 et le méthyle en 21 qui, sans modifier l'aptitude à la fixation sur les sites récepteurs, empêchent ou retardent la métabolisation du produit dans l'organisme par les enzymes réductrices ou hydrogénantes.

Dans la formule générale I, le reste R peut être un radical alcoyle inférieur comme par exemple, un méthyle, un éthyle ou un isopropyle. il peut être un reste acyle dérivé d'un acide alcoyl ou aryl carboxylique comme par exemple le reste acétyle, propionyle, butyryle, pentanoyle, hexanoyle, benzoyle ou napthtoyle. Il peut également dériver d'un acide non saturé comme l'acide crotonique, senecioïque oul'acide 2-méthyl penténoïque. Il peut également dériver d'un acide alcoyl ou cycloalcoyl carbonique comme l'acide cyclohexyl ou cyclopentyl carbonique.

L'invention concerne aussi un procédé de préparation des composés de formule générale I qui consiste essentiellement en ce que l'on soumet un 17-céto 18-méthyl gonadiène II choisi dans le groupe constitué par les 3-alcoxy estra 1,3,5-triènes de formule générale IIa 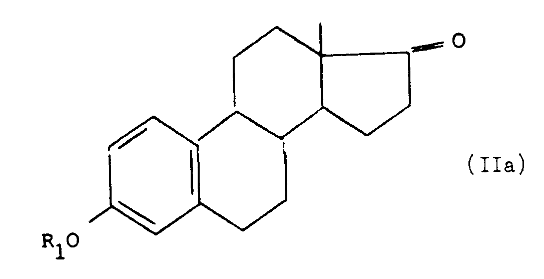
dans laquelle
R₁ représente un radical alcoyle inférieur ayant de 1 à 6 atomes de carbone
et les 3-alcoxy estra 3,5-diènes de formule générale IIb 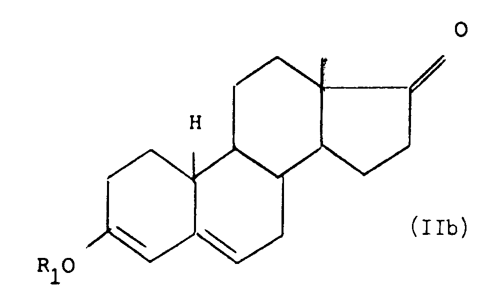
dans laquelle
R₁ a la signification fournie précédemment
à l'action d'un halogénure de (triaryl propyl) phosphonium ou de l'ylide correspondant de formule générale 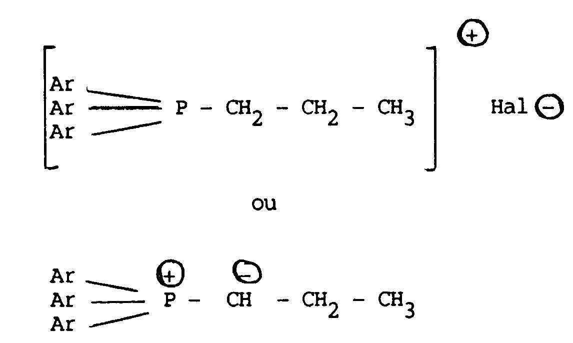
dans laquelle Ar est un radical phényle substitué ou non substitué et Hal est un atome d'halogène autre que le fluor.

pour obtenir un dérivé propylidénique choisi dans le groupe constitué par les dérivés de l'estra 1,3,5-triène de formule générale IIIa 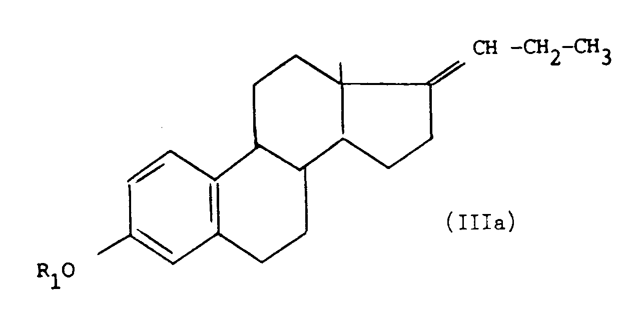
(sous forme d'isomère E ou d'isomère Z)
dans laquelle R₁ est défini comme précédemment
et par les dérivés de l'estra 3,5-diène de formule générale IIIb 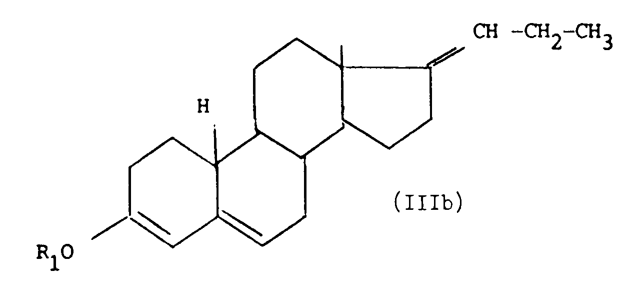
(sous forme d'isomère E ou d'isomère Z)
dans laquelle R₁ est défini comme précédemment
soumet les composés de formule générale IIIa à une hydrogénation selon la méthode de BIRCH pour former un 3-oxo estra 4-ène que l'on convertit en composé de formule générale IIIb par action d'un agent d'alcoylation en milieu acide, fait réagir le composé de formule générale IIIb avec un agent de formylation du type VILSMEIER-HACK pour obtenir le dérivé 6-formylé correspondant de formule générale IV 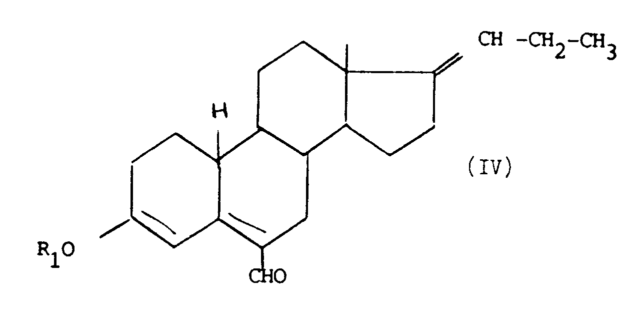
(sous forme d'isomère E ou d'isomère Z)
dans laquelle R₁ est défini comme précédemment
réduit le dérivé 6-formylé à l'aide d'un hydrure mixte de métal alcalin puis déshydrate le dérivé 6-hydroxyméthylé formé en milieu acide pour obtenir le dérivé 3-oxo 6-méthylénique de formule V 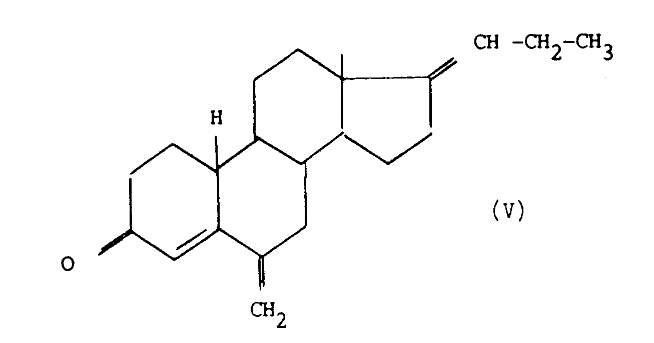
(sous forme d'isomère E ou d'isomère Z)
isomérise ce dernier par réaction avec un catalyseur d'isomérisation pour former le dérivé 3-oxo 6-méthyl estra 4,6-diénique de formule VI 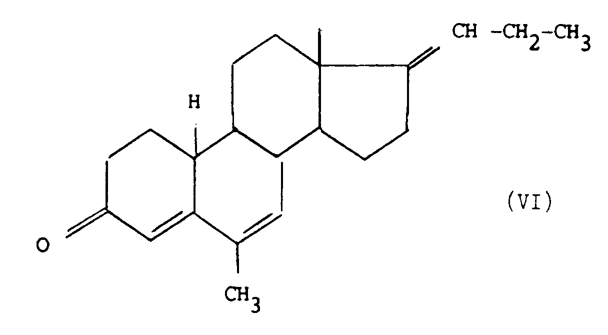
(sous forme d'isomère E ou d'isomère Z)
soumet ce dernier à une bis-hydroxylation à l'aide d'un réactif de bis-hydroxylation formé de tétroxyde d'osmium et d'un hydropéroxyde de N-oxyde en milieu inerte pour former le dérivé 17α-hydroxy 20-cétonique correspondant de formule 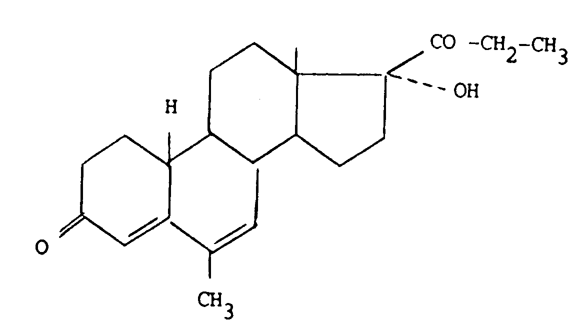
que l'on peut alcoyler par action d'un halogénure d'alcoyle en milieu basique ou tétrahydropyranyler par action du dihydropyran en milieu acide ou acyler par action d'un dérivé fonctionnel d'acide carboxylique ou carbonique en présence d'un réactif acide.

La réaction du composé de formule IIa ou IIb avec l'halogenure de triaryl propylphosphonium ou son ylide conduit principalement à l'isomère Z en 17. Toutefois, l'isomère E formé en proportion moindre peut être isolé par des méthodes physiques et la synthèse peut être poursuivie sans différence notable soit avec l'isomère Z soit avec l'isomère E.

Le procédé selon l'invention peut être également défini par les modes d'exécution suivants, actuellement préférés :
**1**- la réaction du 17-oxogonane de formule IIa ou IIb avec l'halogenure de triaryl propyl phosphonium est effectuée en milieu basique dans un solvant polaire.
**2**- l'agent basique est l'hydrure de sodium, l'isopropylamidure de lithium, le terbutylate de potassium ou l'amidure de sodium.
**3**- le solvant polaire est le dimethylsulfoxyde ou l'hexamethylphosphorotriamide.
**4**- la réduction du composé IIIa selon BIRCH est effectuée par réduction dans l'ammoniac liquide suivie d'un traitement par un acide fort comme l'acide chlorhydrique.
**5**- l'alcoylation du 3-oxo estra 4-ène en composé de formule IIIb est réalisée par action d'un alcanol en milieu acide ou d'un orthoformiate d'alcoyle en milieu acide.
**6**- le réactif de VILSMEIER-HACK est obtenu par action d'oxychlorure de phosphore sur une amine tertiaire comme la diméthylaniline ou un amide di-substitué comme le diméthyl formamide.
**7**- la réduction du dérivé 6-formylé est effectué par un aluminohydrure de métal alcalin ou par un borohydrure de métal alcalin dans un solvant non réactif comme un éther cyclique ou un alcanol.
**8**- la deshydratation du dérivé 6-hydroxyméthylé en dérivé 6-méthylénique est effectuée par traitement par un acide fort comme l'acide chlorhydrique, l'acide perchlorique ou l'acide sulfurique.
**9**- le réactif d'isomérisation est un métal de la famille du platine fixé sur un support inerte comme le palladium sur charbon, le platine sur charbon ou le palladium sur carbonate de calcium.
**10**- la réaction de bis-hydroxylation est effectuée au moyen de tétroxyde d'osmium et d'un hydroperoxyde dérivé d'un N-oxyde d'amine tertiaire comme le N-oxyde de trimethylamine, le N-oxyde de triethylamine ou le N-oxyde de morpholine.
**11**- l'acylation du dérivé 17α-hydroxylé est effectué par action d'un chlorure ou d'un anhydride d'acide en présence d'un acide minéral fort comme l'acide chlorhydrique ou l'acide sulfurique ou d'un acide de Lewis comme le trifluorure de Bore.
**12**- l'alcoylation du dérivé 17α-hydroxylé est effectuée par action du méthoxy méthanol ou du dihydropyran en présence d'acide p.toluène sulfonique dans un solvant inerte.

L'invention s'étend également aux compositions pharmaceutiques renfermant à titre de principe actif, au moins un composé de formule générale I en mélange ou en association avec un excipient ou un véhicule inerte, non toxique, pharmaceutiquement acceptable.

Les compositions pharmaceutiques selon l'invention sont destinées à l'administration par voie parentérale, orale, rectale, per-muqueuse, per cutanée ou pernasale.

Pour l'administration par voie parentérale, les composés de formlule générale I sont administrés sous forme de solutés ou de suspensions injectables conditionnés en ampoules, en flacons multidoses ou en seringues auto-injectables.

Pour l'administration par voie orale, les composés de formule générale I sont administrés sous forme de comprimés nus ou enrobés, de dragées, de gélules, de capsules molles, de pilules, de poudres ou de granulés.

Pour l'administration par voie rectale, les composés de formule générale I sont présentés sous forme de suppositoires ou de capsules rectales.

Pour l'administration par voie per muqueuse, les composés de formule générale I sont présentés sous forme de solutions huileuses, de crèmes, de gelées ou de capsules. Il est possible d'administrer les composés de formule générale I soit par la muqueuse vaginale, soit par la muqueuse nasale sous forme de spray ou de gel.

Pour l'administration par voie per cutanée, les composés de formule générale I sont présentés sous forme de solution ou de crèmes dans un solvant pénétrant comme l'alcool benzylique, le diméthylsulfoxyde ou l'azone.

Ces compositions pharmaceutiques selon l'invention trouvent un emploi en thérapeutique pour le traitement des troubles gynécologiques dûs à une insuffisance lutéale tels que irrégularité menstruelle, dysménorrhée, syndrôme prémenstruel et troubles de la ménopause.

La posologie usuelle s'échelonne de 0,05 mg à 25 mg et la posologie journalière s'échelonne de 0,1 mg à 50 mg en administration continue ou périodique.

L'invention s'étend encore aux produits intermédiaires formés au cours de la synthèse en tant que moyens, à savoir :
- le 6-formyl 3-alcoxy 17(20)propylidène estra 3,5-diène (sous forme d'isomère E ou Z) de formule générale IV
- le 6-méthylène 3-oxo 17(20)propylidène estra 4-ène (sous forme d'isomère E ou Z) de formule V
- le 6-méthyl 3-oxo 17(20)propylidène estra 4,6-diène (sous forme d'isomère E ou Z) de formule VI
Les exemples suivants illustrent l'invention :

### EXEMPLE I

### 6,21-dimethyl 17α-hydroxy 3,20-dioxo 19-nor pregna 4,6-diène

### Stade A : 3-alcoxy 21-méthyl 19-nor pregna 3,5,17(20)-triène

Par le procédé décrit par A.M KRUBINER et coll. dans J. Org. Chem 33 (1968) 1713 au départ de 3-méthoxy 17-céto estra-1,3,5(10)-triène (IV), on obtient successivement le 3-méthoxy 21-méthyl estra 1,3,5(10), 17(20)-tétraene (V) (pF 76°, [α]_{D} +59°) puis le 3-céto 21-méthyl 19-norpregna-4, 17(20)-diène (VI) par réduction selon la méthode de BIRCH-NELSON sous la forme d'une huile fluide, jaune paille ([α] +47°). Ce composé est transformé sous l'action de l'orthoformiate de triéthyle en présence de traces d'acide paratoluène sulfonique au sein de l'éthanol en 3-éthoxy 21-méthyl 19-norpregna-3,5,17(20)-triène (III).

De la même manière le 3-céto 21-méthyl 19-norpregna-4, 17(20)-diène (VI) fournit le 3-méthoxy 21-méthyl 19-norpregna-3,5, 17(20)-triène (VII) avec l'orthoformiate de trimethyle au sein du méthanol en présence de traces d'acide paratoluène sulfonique.
Rendement 79 %, pF = 118°C

### Stade B : 3-ethoxy 6-formyl 21-méthyl 19-norpregnatriène-1,5, 17(20) isomère Z (VIII)

A 30 g de 3-ethoxy 21-méthyl 19-nor pregnatriène-3,5, 17(20) isomère Z (III) et 300 ml de diméthylformamide on ajoute le réactif de Vilsmeier formé de 15.5 ml d'oxychlorure de phosphore et de 124 ml de diméthylformamide à +5°C.

Le milieu réactionnel est maintenu sous agitation pendant 1 h 15. On ajoute 140 ml d'une solution aqueuse saturée d'acétate de sodium, il se forme un précipité jaune cristallin, après 15 mn, on filtre et on lave à l'eau. Après filtration et lavage par de l'eau, on recueille 20,2 g de cristaux jaunes de 3-éthoxy 6-formyl 21-méthyl 19-norpregnatriène-1,5, 17(20) (isomère Z) (VIII).
Rendement 62 %. Le point de fusion du produit pur est F = 99°
Pouvoir rotatoire [α]_{D} = 21.5° (C = 1% dioxanne)

### Stade C : 21-méthyl 6-méthylène 3-oxo 19-norpregna-4, 17(20)-diène, isomère Z (IX)

14 g de 3-ethoxy 6-formyl 21-méthyl 19-norpregna-1,5, 17(20)-triène isomère Z (VIII) et 140 ml de méthanol sont maintenus sous agitation pendant 1 h 20 avec 960 mg de borohydrure de sodium à 0°C. on ajoute ensuite, lentement, 15 ml d'acide chlorhydrique 2N et on agite jusqu'à obtention de cristaux. On obtient 8,7 g de 21-méthyl 6-méthylène 3-oxo 19-norpregna-4, 17(20)diène, isomère Z (IX).
Rendement 71 %. Le point de fusion est 110-114°C
Le pouvoir rotatoire est [α]_{D} = + 218° (1 % dioxanne)
UV max = 261 nm ε = 10600

### Stade C : 6,21-diméthyl 3-oxo 19-norpregna-4,6, 17(20)-triène, isomère Z (X)

3,5 g de 21-méthyl 6-méthylène 3-oxo 19-norpregna-4, 17(20)diène, isomère Z (IX), 3,5 g d'acétate de sodium, 1,4 g de charbon palladié à 5 % dans 350 ml d'éthanol sont portés à reflux pendant 1 h 30. Le milieu réactionnel est filtré, extrait par du chloroforme et lavé à l'eau. Les phases organiques sont chromatographiées sur silice. On recueille dans les éluats 3,1 g de 6,21-diméthyl 3-oxo 19-norpregna-4,6 17(20)-triène isomère Z (X) sous forme d'une huile légèrement jaunâtre.
Rendement 88 %
Le pouvoir rotatoire est [α]_{D} = -31° (1% dioxanne)
UV max = 288 nm ε = 22000

### Stade E : 6,21-diméthyl 17α-hydroxy 3,20-dioxo 19-nor pregna 4,6-diène

2,9 g de 6,21-diméthyl 3-oxo 19-norpregna-4,6, 17(20)-triène (X), 29 ml de terbutanol, 0,58 ml d'une solution de tétroxyde d'osmium à 2,5 % dans le terbutanol et 4,06 g de complexe triéthylamine-N-oxyde hydroperoxyde sont agités pendant 24 h à la température ambiante. On ajoute ensuite 3 g de Célite et 2 g de sulfite de sodium dans l'eau et on agite encore 2 h.

On extrait par le toluène et on percole la phase organique sur un lit de silice pour chomatographie. On évapore l'éluat à sec et on reprend dans le méthanol à chaud. On recueille par cristallisation 1,66 g de 6,21-diméthyl 17α-hydroxy 3,20-dioxo 19-norpregna-4,6-diène.
Rendement 52 %, pF = 204°C

### EXEMPLE II

1 g de 6,21-diméthyl 17α-hydroxy 3,20-dioxo 19-norpregna-4,6-diène, 10 ml de chloroforme, 0.8 ml d'anhydride acétique et 0.15 g d'acide paratoluène sulfonique sont portés à reflux pendant 50 mn. On ajoute 2 ml de méthanol et O.1 ml d'acide chlorhydrique concentré et on porte à nouveau au reflux pendant 1 h. On extrait le milieu avec du chloroforme, on lave par de l'eau, on amène à sec sous vide.

Le résidu est repris pour cristallisation dans le méthanol. On recueille 780 mg de cristaux de 17α-acétoxy 6,21-diméthyl 3,20-dioxo 19-norpregna-4,6-diène.
Rendement 69 %. Spectre UV max 288 nm ε = 23400
Le point de fusion est de 203°C
Pouvoir rotatoire [α]_{D} = -34° (1% dans le dioxanne)
Spectre RMN dans le chloroforme denteré :

| | | |
|---|---|---|
| 0,68 Hz | 3H | |
| | singulet | C18 |
| 1,05 Hz | 3H | |
| | triplet | |
| | J = 7Hz | C22 |
| 1,86 Hz | 3H | |
| | singulet | méthyl en C6 |
| 2,08 Hz | 3H | |
| | singulet | CH₃-CO |
| 5,95 Hz | 1H | |
| | singulet | H en C7 |
| 6,05 Hz | 1H | |
| | singulet | H en C6 |

Selon le même procédé, on a préparé par action du chlorure de propionyle, le 6,21-diméthyl 17α-propionyloxy 19-nor pregna 4,6-diène
- par action du chlorure de caproyle, on obtient le 6,21-diméthyl 17α-caproyloxy 19-norpregna 4,6-diène
- par action du chlorure de trimethylacetyle, on obtient le 6,21-dimethyl 17α-trimethylacétyloxy 19-nor pregna 4,6-diène
- par action du chlorure de benzoyle, on obtient le 6,21-diméthyl 17α-benzoyloxy 19-nor pregna 4,6-diène
- par action du chlorure de monochloracetyle, on obtient le 6,21-diméthyl 17α-chloracetoxy 19-nor pregna 4,6-diène

### EXEMPLE III

### Etude pharmacologique

La liaison aux récepteurs de la progestérone, mesurée sur les récepteurs de l'utérus contre la progestérone marquée, montre pour les composés de l'invention, une affinité de 2,5 fois celle de la progestérone.

### EXEMPLE IV

Comprimés à 2,5 mg de 17α-acétoxy 6,21-diméthyl 3,20-dioxo 19-nor pregna 4,6-diène

| | |
|---|---|
| Principe actif | 2,50 g |
| Lactose | 110 g |
| Amidon de Maïs | 17,5 g |
| Amidon de blé | 8,1 g |
| Carboxyméthyl amidon sodique | 4,5 g |
| Stéarate de Magnesium | 12,4 g |

pour 1.000 comprimés finis au poids moyens de 0,155 g.

## Revendications

1. Les 6,21-diméthyl 19-nor prégnadiènes de formule générale I 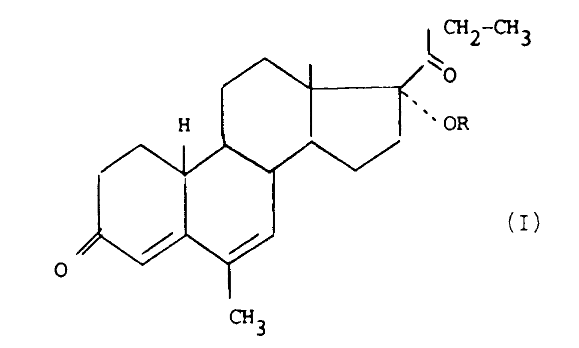 dans laquelle R représente de l'hydrogène, un radical alcoyle inférieur, un radical méthoxy méthyle, un radical tétrahydropyranyle ou le reste acyle d'un acide organique carboxylique ou carbonique ayant de 1 à 10 atomes de Carbone.

2. Un composé selon la revendication 1°, à savoir le 17α-hydroxy 3,20-dioxo 6,21-diméthyl 19-nor pregna 4,6-diène.

3. Un composé selon la revendication 1°, à savoir le 17α-acetoxy 3,20-dioxo 6,21-diméthyl 19-nor pregna 4,6-diène

4. Un composé selon la revendication 1°, à savoir le 17α-tétrahydropyranyloxy 6,21-diméthyl 3,20-dioxo 19-nor pregna 4,6-diène.

5. Un procédé d'obtention des composés de formule générale I 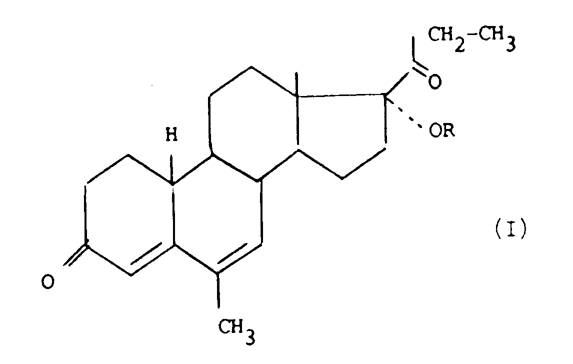 dans laquelle R est défini comme précédemment
caractérisé en ce qu'on soumet un 17-céto 18-méthyl gonadiène II choisi dans le groupe constitué par les 3-alcoxy estra 1,3,5-triènes de formule générale IIa 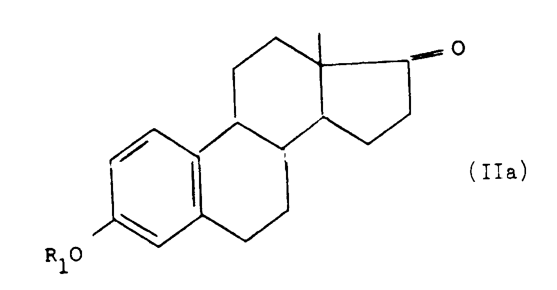 dans laquelle R₁ représente un radical alcoyle inférieur, ayant de 1 à 6 atomes de carbone et les 3-alcoxy estra 3,5-diènes de formule générale IIb 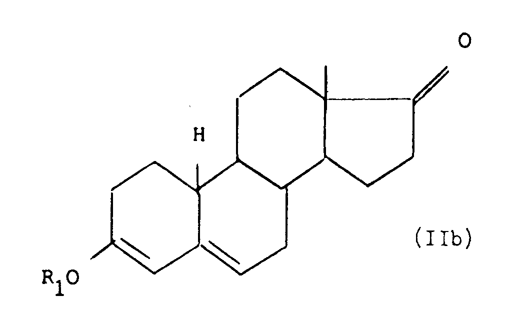 dans laquelle R₁ a la signification fournie précédemment à l'action d'un halogénure de triaryl propyl phosphonium ou de l'ylide correspondant de formule générale 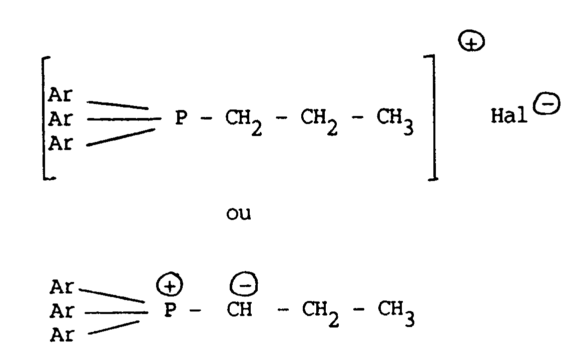 dans laquelle Ar est un radical phényle substitué ou non substitué
et Hal est un atome d'halogène autre que le fluor
pour obtenir un dérivé propylidénique choisi dans le groupe constitué par les dérivés de l'estra 1,3,5-triène de formule générale IIIa 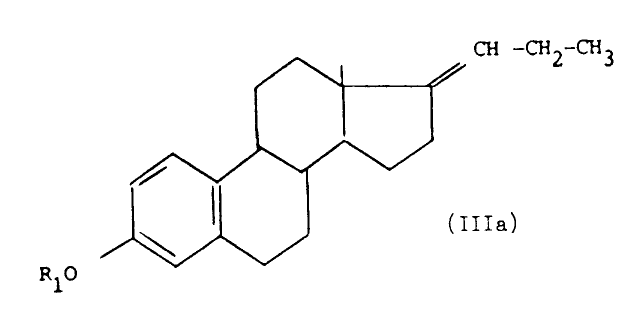 (sous forme d'isomère E ou d'isomère Z)
dans laquelle R₁ est défini comme précédemment
et par les dérivés de l'estra 3,5-diène de formule générale IIIb 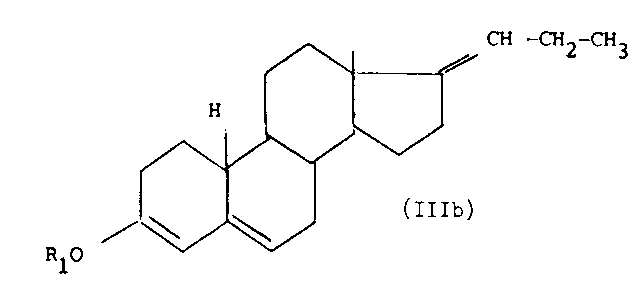 (sous forme d'isomère E ou d'isomère Z)
dans laquelle R₁ est défini comme précédemment
soumet les composés de formule générale IIIa à une hydrogénation selon la méthode de BIRCH pour former un 3-oxo estra 4-ène que l'on convertit en composé de formule générale IIIb par action d'un agent d'alcoylation en milieu acide, fait réagir le composé de formule générale IIIb avec un agent de formylation du type VILSMEIER-HACK pour obtenir le dérivé 6-formylé correspondant de formule générale IV 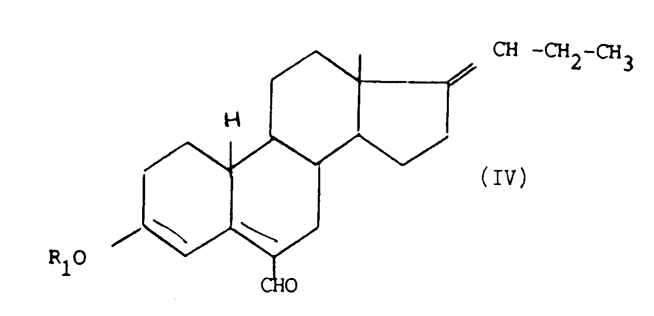 (sous forme d'isomère E ou d'isomère Z)
dans laquelle R₁ est défini comme précédemment
réduit le dérivé 6-formylé à l'aide d'un hydrure mixte de métal alcalin puis traite en milieu acide le dérivé 6-hydroxyméthylé formé pour obtenir le dérivé 3-oxo 6-méthylénique de formule V 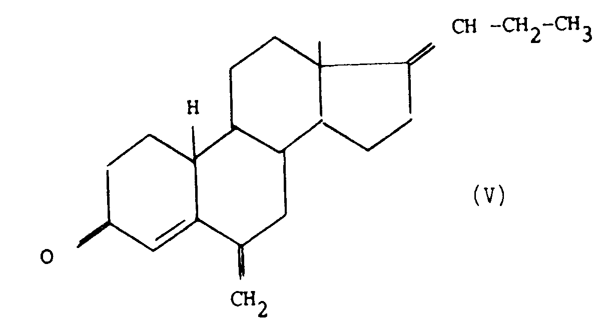 (sous forme d'isomère E ou d'isomère Z)
isomérise ce dernier par réaction avec un catalyseur d'isomérisation pour former le dérivé 3-oxo 6-méthyl estra 4,6-diénique de formule VI 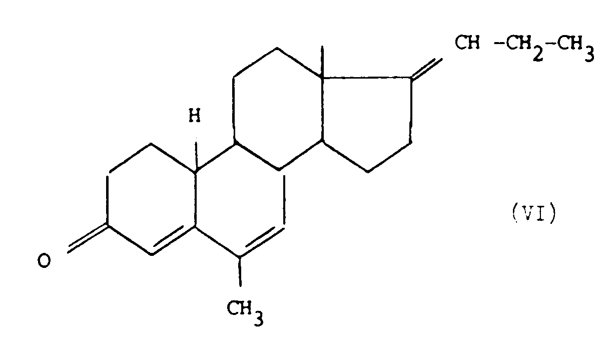 (sous forme d'isomère E ou d'isomère Z)
soumet ce dernier à une bis-hydroxylation à l'aide d'un réactif de bis-hydroxylation formé de tétroxyde d'osmium et d'un hydropéroxyde en milieu inerte pour former le dérivé 17α-hydroxy 20-cétonique correspondant de formule 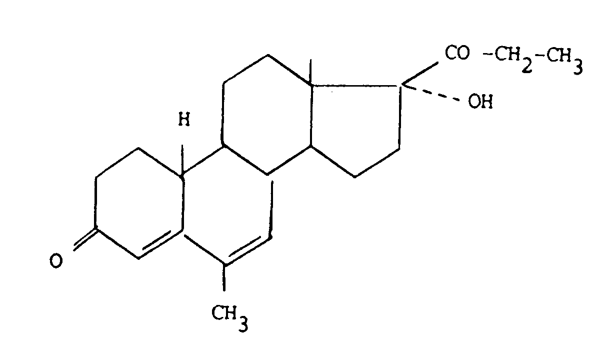 que l'on peut alcoyler par action d'un halogénure d'alcoyle en milieu basique ou tétrahydropyranyler par action du dihydropyran en mieu acide ou acyler par action d'un dérivé fonctionnel d'acide carboxylique ou carbonique en présence d'un réactif acide.

6. Un procédé selon la revendication 5° dans lequel l'halogénure de triarylpropyl phosphonium est le bromure de propyl triphenylphosphonium

7. Un procédé selon la revendication 5 dans lequel le réactif de bis hydroxylation est formé de tétroxyde d'Osmium et d'hydropéroxyde de N-oxyde de Triethylamine

8. Les compositions pharmaceutiques renfermant à titre de principe actif au moins un composé de formule générale I selon la revendication 1°, en association ou en mélange avec un excipient ou un véhicule inerte non toxique pharmaceutiquement acceptable.

9. Une composition pharmaceutique selon la revendication 8° dans laquelle, la quantité de principe actif varie de 0,050 mg à 25 mg par prise unitaire.

10. Une composition pharmaceutique selon la revendication 8 ou la revendication 9 dans laquelle, l'excipient ou le véhicule est un de ceux qui conviennent pour l'administration par voie parentérale, orale, rectale, permuqueuse ou percutanée.

11. En tant que moyens pour l'obtention des composés de l'une des revendication 1 à 4, les 6-formyl 3-alcoxy 17(20)propylidène estra 3,5-diène sous forme d'isomère E ou Z de formule générale IV

12. En tant que moyen pour l'obtention des composés de l'une des revendications 1 à 4, le 6-méthylène 3-oxo 17(20)propylidène estra 4-ène de formule V

13. En tant que moyen pour l'obtention des composés de l'une des revendications 1 à 4, le 6-méthyl 3-oxo 17(20)propylidène estra 4,6-diène de formule VI.

## Claims

1. The 6,21-dimethyl 19-nor pregnadiens of general formula I 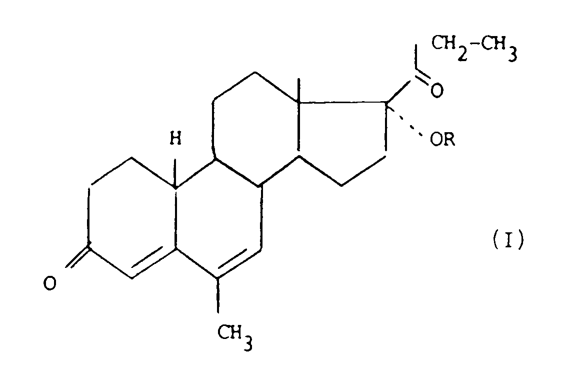 wherein R is a hydrogen, a lower alkyl radical, a methoxymethyl radical, a tetrahydropyranyl radical, or the acyl residue of an organic carboxylic or carbonic acid having from 1 to 10 carbon atoms.

2. A compound according to claim 1° which is 17α-hydroxy 3,20-dioxo 6,21-dimethyl 19-nor pregna 4,6-dien.

3. A compound according to claim 1° which is 17α-acetoxy 3,20-dioxo 6,21-dimethyl 19-nor pregna 4,6-dien.

4. A compound according to claim 1° which is 17α-tetrahydropyranyloxy 6,21-dimethyl 3,20-dioxo 19-nor pregna 4,6-dien.

5. A process for producing the compounds of general formula I : 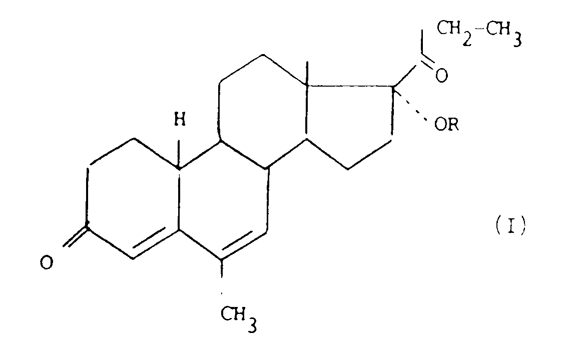 wherein R is defined as previously given
characterised in that a 17-keto 18-methyl gonadien II selected from the group consisting of the 3-alkoxy estra 1,3,5-triens of general formula IIa : 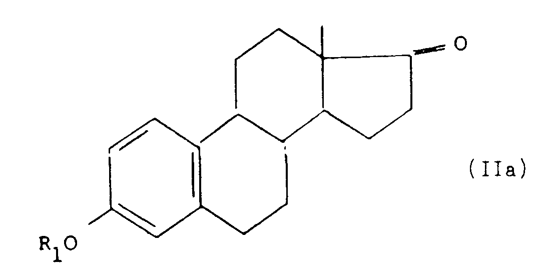 wherein R₁ represents a lower alkyl radical having from 1 to 6 carbon atoms
and the 3-alkoxy estra 3,5-diens of general formula IIb : 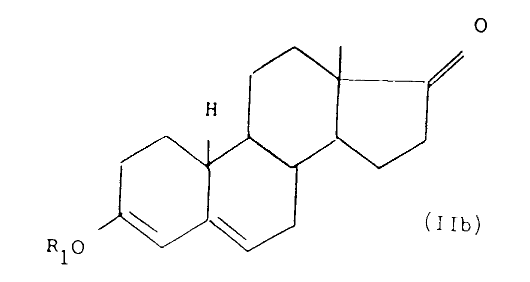 wherein R₁ has the previously given definition
is submitted to the action of a triaryl propyl phosphonium halide or of the ylide thereof having the general formula : 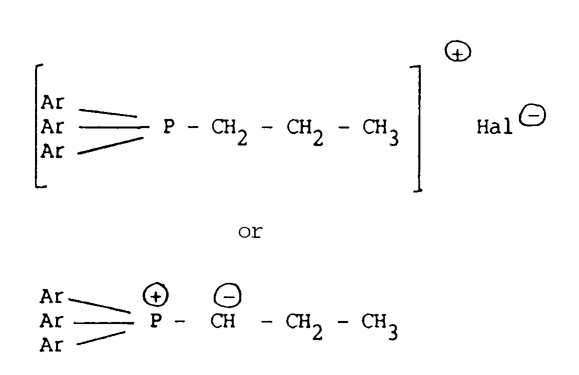 wherein Ar is an unsubstituted or substituted phenyl group
and Hal is a halogen atom other than fluorine
to produce a propylidenic derivative selected from the group consisting of derivatives of estra 1,3,5-trien of general formula IIIa 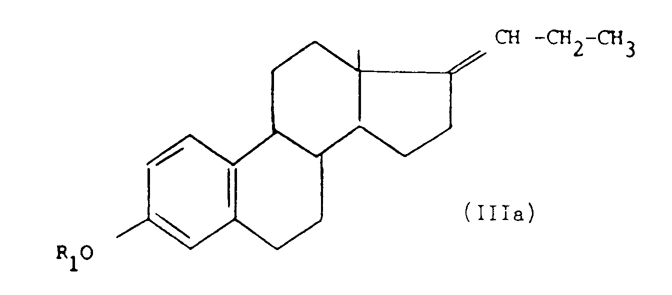 (in the form of E isomer or Z isomer)
wherein R₁ is defined as previously given
and derivatives of estra 3,5-diens having the general formula IIIb : 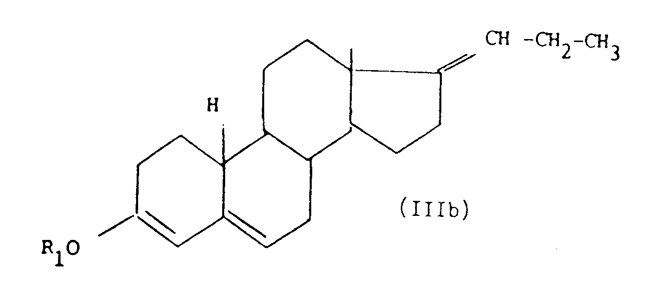 (in the form of E isomer or Z isomer)
wherein Z is defined as previously
submit the latter compounds of formula IIIa to a hydrogenation according to the BIRCH's method to produce a 3-oxo estra 4-en which is converted into a compound of formula IIIb by means of an alkylating agent in acidic medium, let react the compound of general formula IIIb with a formylating agent of the VILSMEIER-HACK's type to produce a 6-formylated derivative of general formula IV : 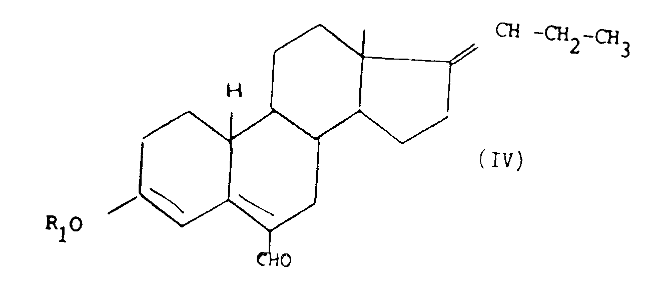 (in the form of E isomer or Z isomer)
wherein R₁ is defined as above given
reduce the 6-formylated derivative by means of a alkali metal mixed hydride then treats in acidic medium the thus formed 6-hydroxymethylated derivative to produce the 3-oxo 6-methylen derivative of formula V : 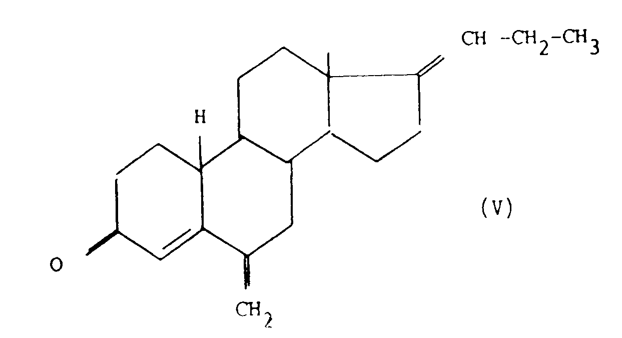 (in the form of E-isomer or Z-isomer)
isomerizes the latter by reacting it with an isomerization catalyst to produce a 3-oxo 6-methyl estra 4,6-dien derivative of formula VI : 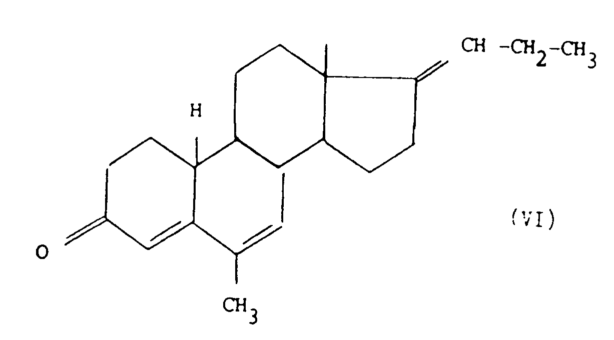 (in the form of E-isomer or Z-isomer)
submit the latter to a bis-hydroxylation by means of a bis-hydroxylating agent consisting of osmium tetroxide and a hydroperoxide, in an inert medium to produce the corresponding 17α-hydroxy 20-keto derivative of formula : 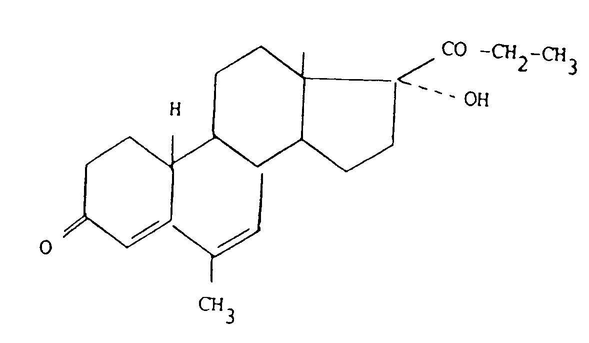 which may be alkylated by means of an alkyl halide in basic medium or tetrahydropyranylated by means of dihydropyran in acidic medium or acylated by means of a functional derivative of a carboxylic or carbonic acid in the presence of an acid reagent.

6. A process according to claim 5° wherein the triarylpropyl phosphonium halide is propyl triphenylphosphonium bromide.

7. A process according to claim 5° wherein the bis hydroxylating agent consists of Osmium tetroxide and Triethylamine N-oxide hydroperoxide.

8. The pharmaceutical compositions containing as active ingredient at least one compound of general formula I according to claim 1°, in admixture or conjunction with an inert non-toxic pharmaceutically acceptable carrier or vehicle.

9. A pharmaceutical composition according to claim 8° wherein the amount of active ingredient ranges from 0.050 mg to 25 mg per unit dosage.

10. A pharmaceutical composition according to claim 8° or claim 9° wherein the carrier or vehicle is one of those suitable for parenteral, oral, rectal permucous or percutaneous ways of administration.

11. As means for the production of a compound according to anyone of claims 1 to 4, the 6-formyl 3-alkoxy 17(20)propyliden estra 3,5-dien in the form of E or Z isomer having the general formula IV.

12. As a mean for the production of the compounds according to anyone of claims 1 to 4, 6-methylen 3-oxo, 17(20)propyliden estra 4-en of formula V.

13. As a mean for the production of the compounds according to anyone of claims 1 to 4, 6-methyl 3-oxo 17(20)propyliden estra 4,6-dien of formula VI.

## Patentansprüche

1. Die 6,21-Dimethyl 19-nor pregnadiene der allgemeinen Formel I 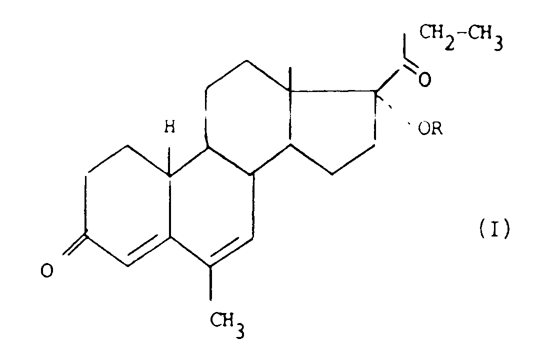 worin R für einen Wasserstoff, einen niedrig Alkyl Radikal, einen Methoxymethyl Radikal, einen Tetrahydropyranyl Radikal, oder den Acyl Rest einer organischen Carbon- oder Kohlensäure, mit 1 bis 10 Kohlenstoff Atomen, steht.

2. Eine Verbindung nach Anspruch 1° d.h. 17α-Hydroxy 3,20-dioxo 6,21-dimethyl 19-nor pregna 4,6-dien.

3. Eine Verbindung nach Anspruch 1° d.h. 17α-Acetoxy 3,20-dioxo 6,21-dimethyl 19-nor pregna 4,6-dien.

4. Eine Verbindung nach Anspruch 1° d.h. 17α-Tetrahydropyranyloxy 6,21-dimethyl 3,20-dioxo 19-nor pregna 4,6-dien.

5. Verfahren zur Herstellung der Verbindungen der allgemeinen Formel I : 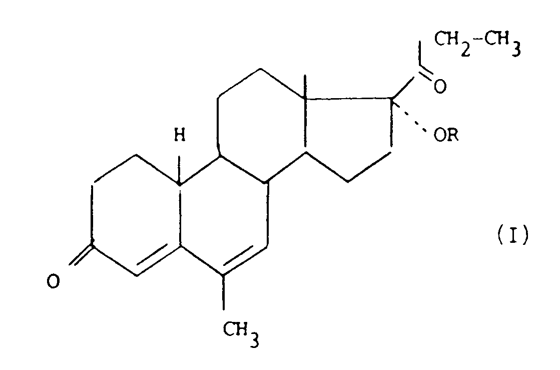 worin R wie vorher definiert ist
dadurch gekennzeichnet ist, dass ein 17-Keto 18-methyl gonadien II aus der Gruppe bestehend aus den 3-Alkoxy estra 1,3,5-Trienen der allgemeinen Formel IIa : 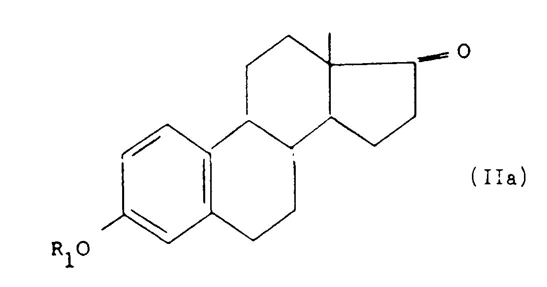 worin R₁ fur einen niedrigen Alkyl Radikal mit 1 bis 6 Kohlenstoff Atomen steht
und den 3-Alkoxy estra 3,5-dienen der allgemeinen Formel IIb : 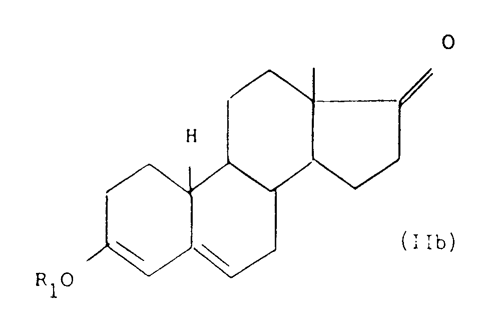 worin R1 die vorherige Bedeutung besitzt,
die Einwirkung eines Triarylpropylphosphonium Halogenids oder dessen entsprechenden Ylid, der allgemeinen Formel : 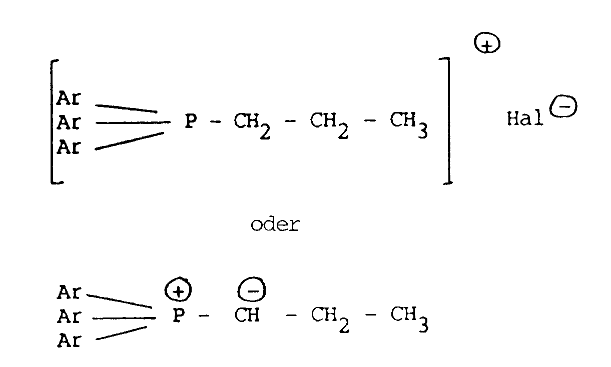 worin Ar ein substituiertes oder unsubstituiertes Phenyl Radikal ist
und Hal ein Halogen Atom ausser als Fluor ist,
unterwirft, um ein propylidenische Derivat aus der Gruppe bestehend aus den Derivaten von Estra 1,3,5-Trien der allgemeinen Formel IIIa : 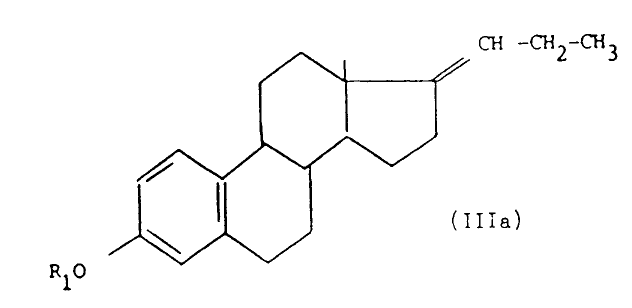 (als E-Isomer oder Z-Isomer)
worin R₁ as vorher definiert ist
und den Derivaten von Estra 3,5-dien der allgemeinen Formel IIIb : 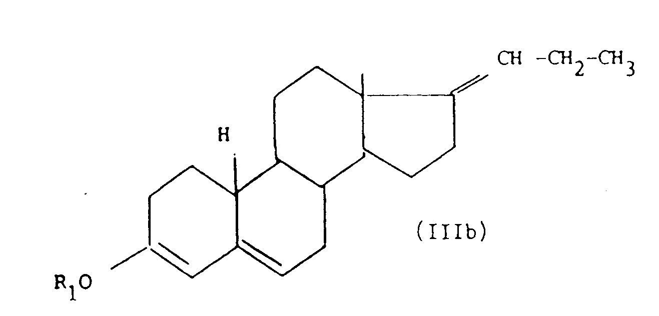 (als E-Isomer oder Z-Isomer)
worin R₁ wie vorher definiert ist
ausgewählt ist
dass die Verbindungen der allgemeinen Formel IIIa zu eine nach der BIRCH's Methode, Hydrierung einwirken lässt um ein 3-Oxo estra 4-en zu gewinnen, das in einer Verbindung der allgemeinen Formel IIIb mittels eines Alkylierungs Mittel in Saüre Medium überführt, dass die Verbindung der allgemeinen Formel IIIb mit einer Formylierungsmittel des VILSMEIER-HACK Typs, reagieren lässt um das entsprechede 6-Formyl Derivat der allgemeinen Formel IV 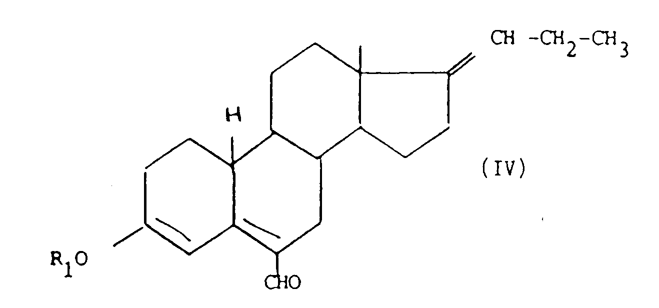 (als E oder Z Isomer)
worin R₁ wie vorher definiert ist
zu gewinnen, dass man das 6-Formyl Derivat mittels eines Alkali metall Mischhydrid reduziert, denn man in Säure Medium das so erhaltene 6-Hydroxymethyl Derivat behandelt um das 3-oxo 6-methylen Derivat der allgemeinen Formel V : 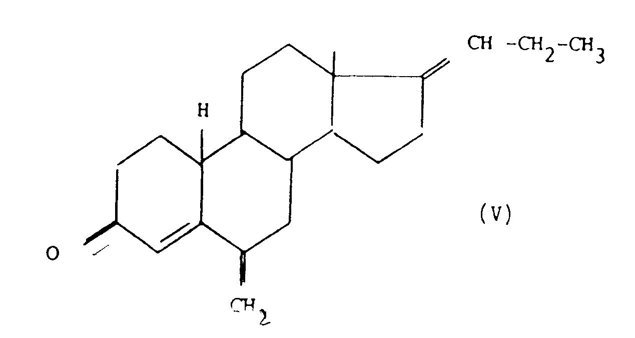 (als E oder Z Isomer)
zu herstellen
man dieses durch Reaktion mit einem Isomerisierungs Katalyst isomerisiert um eine 3-oxo 6-methyl estra 4,6-dienische Derivat der Formel VI 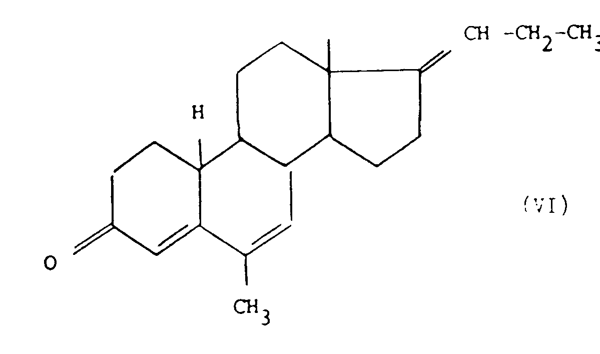 (als E oder Z Isomer)
zu gewinnen
das letzeres zu einer Bis-hydroxylierung mittels eines Bishydroxylierungsmittels aus Osmium Tetroxid und eines Hydroperoxid bestehende Reagenz in einem inerten Medium, unterwirft, um das entsprechede 17α-Hydroxy 20-Ketonische Derivat der allgemeinen Formel 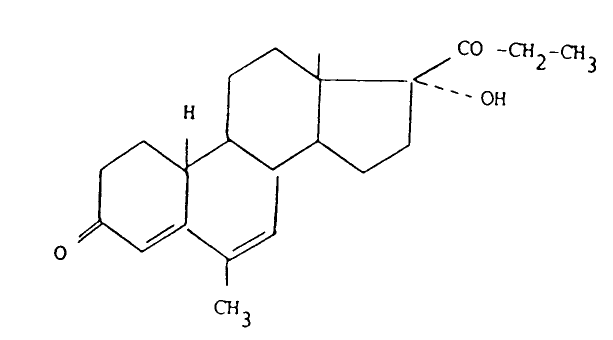 zu herstellen
das man durch die Einwirkung eines Alkyl Halogenids in basichen Medium alkylieren kann oder mittels der Einwirkung von Dihydropyran in Säure Medium tetrahydropyranylieren lässt oder mittels der Einwirkung eines funktionellen Derivat von einer Carbon-oder Kohlen Säure in Gegenwart eines acidischen Reagenz acyliert lässt.

6. Verfahren nach Anspruch 5° worin das Triarylpropylphosphonium Halogenid das Propyl Triphenyl phosphonium Bromid ist.

7. Verfahren nach Anspruch 5° worin das bis-hydroxylierung Reagenz aus Osmium Tetroxid und Triethylamin N-oxid Hydroperoxid ausgebildet wird.

8. Pharmazeutische Zubereitungen die als Wirkstoffe mindenstens eine Verbindung der allgemeinen Formel I nach Anspruch 1°, in Verbindung oder Vermischen mit einen inerten ungiftigen pharmazeutisch vertraglischen Träger oder Vehikel, enthalten.

9. Eine pharmazeutische Zubereitung nach Anspruch 8° worin die Menge an Wirkstoff von 0,050 mg bis 25 mg bei einzelne Dosis schwänkt.

10. Eine pharmazeutische Zubereitung nach Anspruch 8° oder Anspruch 9° worin der Träger oder der Vehikel ein der solche die fur parenterale, orale, rektale, permukale oder percutan Abreichungsweg geeignet sind.

11. Als Mittel für die Gewinnung der Verbindungen nach einer der Ansprüche 1 bis 4, die 6-Formyl 3-alkoxy 17(20)-propyliden estra 3,5-diene in der Form von E isomer oder Z-isomer, der allgemeinen Formel IV.

12. Als Mittel für die Gewinnung der Verbindungen nach einer der Ansprüch 1° bis 4, das 6-Methylen 3-oxo 17(20)-propyliden estra 4-en der Formel V.

13. Als Mittel für die Gewinnung der Verbindungen nach einer der Ansprüche 1° bis 4, das 6-Methyl 3-oxo 17(20)-propyliden estra 4,6-dien der Formel VI.
